# EUROPEAN PATENT SPECIFICATION

(11) **EP 4 379 381 B1**
(45) Date of publication and mention of the grant of the patent: **12.11.2025**
(21) Application number: 22893985.6
(22) Date of filing: 20.04.2022
(51) Int. Cl.: G01N 33/564, G01N 33/58, G01N 33/577, G01N 33/543, G01N 33/68

(54) **MOLECULAR MARKER FOR DIAGNOSING PRIMARY BILIARY CHOLANGITIS AND APPLICATION THEREOF**
MOLEKULARER MARKER ZUR DIAGNOSE DER PRIMÄREN BILIÄREN CHOLANGITIS UND DESSEN ANWENDUNG
MARQUEUR MOLÉCULAIRE POUR LE DIAGNOSTIC DE LA CHOLANGITE BILIAIRE PRIMITIVE ET SON APPLICATION

(30) Priority: 19.11.2021 CN 202111375805
(43) Date of publication of application: 05.06.2024
(73) Proprietor: The First Hospital Affiliated to Pla Army Medical University, Chongqing 400038 (CN); Xiangya Hospital, Central South University, Changsha, Hunan 410008 (CN)
(72) Inventor: CHAI, Jin, Chongqing 400038 (CN); WANG, Huiwen, Changsha, Hunan 410008 (CN); LI, Qian, Chongqing 400038 (CN); PAN, Qiong, Chongqing 400038 (CN); LIAO, Min, Chongqing 400038 (CN); XIAO, Jintao, Changsha, Hunan 410008 (CN); GUO, Yan, Changsha, Hunan 410008 (CN); MAO, Xiuru, Changsha, Hunan 410008 (CN); PENG, Shifang, Changsha, Hunan 410008 (CN); LIU, Xiaowei, Changsha, Hunan 410008 (CN); TANG, Cane, Changsha, Hunan 410008 (CN)
(74) Representative: Bayramoglu et al.
(86) International application number: PCT/CN2022/087926
(87) International publication number: WO 2023/087616

(56) References cited:
- WO-A1-89/05976
- CN-A- 102 435 733
- CN-A- 103 044 548
- CN-A- 106 153 893
- CN-A- 109 085 344
- CN-A- 114 088 939
- US-A1- 2007 141 633
- US-A1- 2012 183 981
- QI XUANCHANG ET AL: "Reduced expression of polymeric immunoglobulin receptor (pIgR) in nasopharyngeal carcinoma and its correlation with prognosis", TUMOR BIOLOGY, KARGER, BASEL, CH, vol. 37, no. 8, 24 February 2016 (2016-02-24), pages 11099 - 11104, XP036039388, ISSN: 1010-4283, [retrieved on 20160224], DOI: 10.1007/S13277-016-4791-X
- YAMAGIWA SATOSHI ET AL: "Autoantibodies in primary biliary cirrhosis: Recent progress in research on the pathogenetic and clinical significance", vol. 20, no. 10, 1 January 2014 (2014-01-01), CN, pages 2606, XP093196596, ISSN: 1007-9327, Retrieved from the Internet <URL:https://www.ncbi.nlm.nih.gov/pmc/articles/PMC3949269/pdf/WJG-20-2606.pdf> DOI: 10.3748/wjg.v20.i10.2606
- GRANITO ALESSANDRO ET AL: "Autoantibodies to speckled protein family in primary biliary cholangitis", vol. 17, no. 1, 31 March 2021 (2021-03-31), London, UK, XP093196597, ISSN: 1710-1492, Retrieved from the Internet <URL:http://link.springer.com/article/10.1186/s13223-021-00539-0/fulltext.html> DOI: 10.1186/s13223-021-00539-0
- SEBODE MARCIAL ET AL: "Autoantibodies in Autoimmune Liver Disease-Clinical and Diagnostic Relevance", FRONTIERS IN IMMUNOLOGY, vol. 9, 27 March 2018 (2018-03-27), Lausanne, CH, XP093196599, ISSN: 1664-3224, DOI: 10.3389/fimmu.2018.00609

## Description

### TECHNICAL FIELD

The present invention relates to the technical field of molecular biology, particularly, to a serum anti-human polymeric immunoglobulin receptor antibody, and more particularly, to a serum anti-human polymeric immunoglobulin receptor antibody as a molecular marker for diagnosing primary biliary cholangitis and use thereof.

### BACKGROUND

Primary biliary cholangitis (PBC) is a chronic cholestatic liver disease. It is characterized by the autoimmune destruction of small and medium-sized intrahepatic bile ducts, which leads to progressive cholestasis, and may slowly progress to cirrhosis and liver failure. At present, there are more than 100 thousand new PBC diagnoses worldwide each year, which are mainly in females, and the morbidity may be up to 0.1% in females aged over 40 years. Previous studies have shown that a variety of factors, such as genetic and environmental factors, immune dysregulation, and the like, are involved in the pathogenesis of PBC. Among these, the loss of the mitochondrial antigen immune tolerance and subsequent autoimmune responses involving humoral and cellular immunities play crucial roles in the pathogenesis of PBC. The autoimmunity of PBC is characterized in that the loss of tolerance of B cells to mitochondrial pyruvate dehydrogenase complex E2 subunit (PDC-E2) results in the production of antimitochondrial antibodies (AMAs) and the activation of autoreactive CD4+ and CD8+ T cells, and a biliary epithelial cell becomes the target of immune attack. However, some patients with PBC are negative for an AMA-M2 subtype, indicating that the PBC is not always dependent on AMA-mediated specific immune response. In addition, since PDC-E2 is also widely expressed in hepatocytes, it is not clear so far why the autoimmune responses preferentially destroy the small and medium-sized intrahepatic bile ducts in patients with PBC, regardless of the presence or absence of AMAs.

As a member of the Fc receptor family, human polymeric immunoglobulin receptor is a core member of the mucosal immune system. It is widely expressed in mucosal epithelial cells, of which the expression is usually upregulated by pro-inflammatory cytokines in viral or bacterial infections. Human polymeric immunoglobulin receptor can specifically bind to polymeric IgA and polymeric IgM, and transports them from the basement membrane of epithelial cells to the apical membrane by transcellular transport and ultimately into exocrine secretion, forming the first line of defense against infections. In recent years, many studies have shown that the high expression of the human polymeric immunoglobulin receptor in the liver is a risk factor for liver fibrosis, which may be associated with hepatitis virus infection and hepatic stellate cell transdifferentiation. The human polymeric immunoglobulin receptor can also promote the metastasis of human hepatocellular carcinoma (HCC). However, whether human polymeric immunoglobulin receptor participates in the development and progression of PBC and whether anti-human polymeric immunoglobulin receptor antibodies are valuable in the diagnosis of PBC have not been reported so far, and the indicators are not clinically applied. CN114088939A discloses exosomal polymeric immunoglobulin receptor as a biomarker for PBC.

Serum AMAs and the M2 subtype thereof are important auxiliary diagnosis indicators of PBC in clinical practice at present. However, there are certain proportions of false positives and false negatives in diagnoses using these indicators. As such, the diagnosis of PBC still mainly depends on liver tissue biopsy. For the early diagnosis of PBC in clinical practice, to find a diagnosis method with simple and reliable sampling procedures and sensitive molecular indicators has become an urgent problem to be solved.

### SUMMARY

Herein is described the detection of the expression and positioning of human polymeric immunoglobulin receptors in liver tissues of AMA-M2-positive PBC patients and AMA-M2-negative PBC patients, control (CTR) subjects, obstructive cholestasis (OC) patients, secondary sclerosing cholangitis (SSC) patients and nonalcoholic steatohepatitis (NASH) patients by using multiplex immunohistochemical fluorescence staining and taking cholangiocyte marker cytokeratin 19 (CK19) as the reference. It has been found that human polymeric immunoglobulin receptors are all expressed in cholangiocytes of the CTR subjects and the OC, SSC and NASH patients, but the expression of human polymeric immunoglobulin receptors in small and medium-sized bile ducts in the livers of the PBC patients is significantly reduced, regardless of AMA-M2 positive or negative. Based on the findings, it is speculated that human polymeric immunoglobulin receptor may be a specific antigen mediating the immunological damage in the pathogenesis of PBC. The serum of the CTR subjects and the PBC and OC patients were detected by using an enzyme-linked immunosorbent assay (ELISA), demonstrating that the anti-human polymeric immunoglobulin receptor antibody in the serum of the PBC patients, both AMA-M2 positive and negative, is significantly increased. This finding suggests that the anti-human polymeric immunoglobulin receptor antibody mediates the damage of cholangiocytes by targeting the human polymeric immunoglobulin receptor antigen on the small and medium-sized bile ducts in the liver, and serum anti-human polymeric immunoglobulin receptor antibodies can thus be used as a molecular marker for diagnosing PBC. At present, anti-AMA-M2 antibody positive is one of the criteria for diagnosing PBC in clinical practice, which easily results in false negative in anti-AMA-M2 antibody negative PBC patients. Therefore, the detection of the anti-human polymeric immunoglobulin receptor antibody is conducive to diagnosing PBC, particularly in AMA-M2 negative PBC patients.

In view of the absence of reliable diagnostic methods with small trauma to patients, and particularly in case of the false negative or false positive in anti-AMA-M2 antibody negative PBC patients, the present invention is intended to provide a molecular marker for diagnosing PBC and use thereof, i.e., a serum anti-human polymeric immunoglobulin receptor antibody as a molecular marker for diagnosing PBC.

In order to achieve the above objective, the present invention adopts the following technical solutions:
Provided is a use of anti-human polymeric immunoglobulin receptor antibody in a sample obtained from a patient as a molecular marker for diagnosing primary biliary cholangitis.

Furthermore, the sample includes an anti-AMA-M2 antibody-positive PBC sample and an anti-AMA-M2 antibody-negative sample, and the sample is a human serum sample.

Furthermore, a reagent is used for detecting the expression level of the anti-human polymeric immunoglobulin receptor antibody.

Furthermore, the reagent for diagnosing a PBC patient is an ELISA kit. The ELISA kit comprises a plate, a standard, a sample diluent, a mouse anti-human polymeric immunoglobulin monoclonal antibody labeled with horseradish peroxidase (detection antibody-HRP), a 20× washing buffer, a substrate A, a substrate B, a stop solution, a sealing film, and a re-sealable bag.

Furthermore, using the ELISA kit comprises:
separating a serum from a whole blood sample as a detection sample;
setting standard wells and sample wells, adding 50 µL of standards of different concentrations into the standard wells, and adding 50 µL of the detection sample into the sample wells; adding nothing into blank wells;
adding 100 µL of the detection antibody (the mouse anti-human polymeric immunoglobulin monoclonal antibody)-HRP into each of the standard wells and the sample wells, sealing the reaction wells with the sealing film, and incubating at 37 °C for 60 min;
washing the plate with a washing solution 5 times;
adding the substrates A and B, each 50 µL, into each well and incubating at 37 °C away from light for 15 min;
adding 50 µL of the stop solution into each well, and measuring optical density (OD) values of the wells at a wavelength of 450 nm within 15 min; and
plotting a standard curve of the measured OD values of the standard vs the concentration of the standard, obtaining a linear regression equation, and introducing the OD value of the sample into the equation to calculate the concentration of the sample.

Compared with the prior art, the present invention has the following beneficial effects:
1. It is found that anti-human polymeric immunoglobulin receptor antibodies can be used as a biomarker for diagnosing PBC. Serum AMA-M2 is a specific indicator for diagnosing PBC, with about 90-95% of the PBC patients being AMA-M2 positive. based on AMA-M2 positive or negative, PBC can be divided into two subtypes of AMA-M2 positive PBC and AMA-M2 negative PBC. For AMA-M2 negative PBC, there's an absence of specific diagnostic markers in clinical practice, which frequently leads to false negative. It is found that the level of the anti-human polymeric immunoglobulin receptor antibody in the serum of PBC patients (both AMA-M2 positive or negative) is significantly increased, providing a new marker for diagnosing PBC, particularly in AMA-M2 negative PBC patients.
2. The biomarker, or anti-human polymeric immunoglobulin receptor antibody, for diagnosing PBC disclosed by the present invention is acquired from the peripheral blood of subjects in clinical application, featuring an easy and non-invasive sampling process and ease to popularize.
3. The present invention discloses a common pathogenesis in AMA-M2 positive and negative PBC patients, i.e., the damage of the small and medium-sized intrahepatic bile ducts mediated by anti-human polymeric immunoglobulin receptor antibodies, which can greatly reduce the false negative and false positive of PBC, particularly the false negative and false positive in AMA-M2 negative PBC patients.

### BRIEF DESCRIPTION OF THE DRAWINGS

In order to more clearly illustrate the technical solutions in the embodiments of the present invention or in the prior art, the drawings required to be used in the description of the embodiments or the prior art are briefly introduced below. It is obvious that the drawings in the description below are some embodiments of the present invention, and those of ordinary skills in the art can obtain other drawings according to the drawings provided herein without creative efforts.
FIG. 1 illustrates the expression and positioning of human polymeric immunoglobulin receptor in the liver of a control subject confirmed by multiplex immunohistochemical fluorescence staining;
FIG. 2 illustrates the expression and positioning of human polymeric immunoglobulin receptor in the liver of an AMA-M2 positive primary biliary cholangitis patient confirmed by the multiplex immunohistochemical fluorescence staining;
FIG. 3 illustrates the expression and positioning of human polymeric immunoglobulin receptor in the liver of an AMA-M2 negative primary biliary cholangitis patient confirmed by the multiplex immunohistochemical fluorescence staining;
FIG. 4 illustrates the expression and positioning of human polymeric immunoglobulin receptor in the liver of an obstructive cholestasis patient confirmed by immunofluorescence staining;
FIG. 5 illustrates the expression and positioning of human polymeric immunoglobulin receptor in the liver of a secondary sclerosing cholangitis patient confirmed by immunofluorescence staining;
FIG. 6 illustrates the expression and positioning of human polymeric immunoglobulin receptor in the liver of a nonalcoholic steatohepatitis patient confirmed by immunofluorescence staining;
FIG. 7 illustrates the level of anti-human polymeric immunoglobulin receptor antibody in the serum of the control subject, the primary biliary cholangitis patient, and the obstructive cholestasis patient determined by enzyme-linked immunosorbent assay.

### DETAILED DESCRIPTION OF THE EMBODIMENTS

The related techniques in the present invention will be clearly and completely described below with reference to the embodiments of the present invention and the drawings of the specification, and it is obvious that the described embodiments are only a part of the embodiments of the present invention but not all of them. Based on the examples of the present invention, all other examples obtained by those of ordinary skills in the art without creative effort shall fall within the protection scope of the present invention.

In the following examples, the liver tissues and serum samples of study objects were acquired from the Southwest Hospital of Army Medical University (AMU), and the sample acquisition had been approved by the Ethics Committee of the hospital.

Example 1: Confirmation of expression and positioning of human polymeric immunoglobulin receptors in liver tissues of CTR subjects and PBC, OC, SSC and NASH patients.

One liver tissue sample was collected from CTR subjects and PBC, OC, SSC and NASH patients of the Southwest Hospital by surgical resection or pathological examination. After paraformaldehyde fixation, dehydration and transparency, and wax embedding, the liver tissue samples were sectioned at a thickness of 4 µm. The expression and positioning of human polymeric immunoglobulin receptor in the liver tissues of the subjects were detected by multiplex immunohistochemical fluorescence staining. The reagents were from a multiplex immunohistochemical fluorescence kit (Absin, abs50014). The specific procedures are as follows:
(1) Dewaxing and hydration: The paraffin sections were incubated in an oven at 65 °C for 1 h, and soaked thrice in fresh xylene for 5 min, in ethanol with concentration gradients, twice in 100% ethanol for 10 min, and twice in 95% ethanol for 10 min. The sections were then washed with sterile water twice for 5 min.
(2) Antigen retrieval: The samples were treated in Tris-EDTAin a microwave oven at high power level for 2 min and at mid/high power level for 13 min, let stand for about 30 min at room temperature, and soaked in sterile water for 3 min and in TBST for 3 min.
(3) Endogenous peroxidase blocking: An endogenous peroxidase blocker (ZSGB-Bio, SP-9000) was dropwise added. The samples were incubated at room temperature for 10 min, and soaked twice in TBST for 3 min.
(4) Blocking: The samples were incubated in 8% goat serum (Absin, abs933) at room temperature for 10 min.
(5) Co-incubation with primary antibody: An anti-cytokeratin 19 antibody (Abcam, ab52625) was diluted with 8% goat serum in a factor of 1:500, and the samples were co-incubated with the antibody in moisture at room temperature for 1 h. The slides were soaked twice in TBST for 3 min.
(6) A secondary antibody working solution was equilibrated to room temperature during the co-incubation with the primary antibody.
(7) Co-incubation with secondary antibody: The secondary antibody working solution was dropwise added until the samples were submerged completely. The samples were incubated in moisture at room temperature for 10 min, and soaked twice in TBST for 3 min.
(8) Fluorescent staining signal amplification: An HRP 520 dye was diluted with a signal amplifier solution in a factor of 1:100, and dropwise added to the slide with a pipettor until the samples were submerged completely. The samples were incubated in moisture at room temperature for 10 min. The slides were then soaked thrice in TBST for 3 min at room temperature.
(9) Antigen retrieval: The samples were treated in Tris-EDTAin a microwave oven at high power level for 2 min and at mid/high power level for 13 min, let stand for about 30 min at room temperature, and soaked in sterile water for 3 min and in TBST for 3 min.
(10) Blocking: The samples were incubated in 8% goat serum at room temperature for 10 min.
(11) Co-incubation with primary antibody: An anti-human polymeric immunoglobulin receptor antibody (Proteintech, 22024-1-AP) was diluted with 8% goat serum in a factor of 1:500, and the samples were co-incubated in moisture at room temperature for 1 h and soaked twice in TBST for 3 min. A secondary antibody working solution was equilibrated to room temperature during the co-incubation with the primary antibody.
(12) Co-incubation with secondary antibody: The secondary antibody working solution was dropwise added until the samples were submerged completely. The samples were incubated in moisture at room temperature for 10 min, and soaked twice in TBST for 3 min.
(13) Fluorescent staining signal amplification: An HRP 570 dye was diluted with a signal amplifier solution in a factor of 1:100, and dropwise added to the slide with a pipettor until the samples were submerged completely. The samples were incubated in moisture at room temperature for 10 min. The slides were then soaked thrice in TBST for 3 min at room temperature.
(14) Nucleus staining and mounting: A 1× DAPI (diluted with ddH₂O in a factor of 1:100) working solution was dropwise added to the samples. The samples were incubated at room temperature for 5 min, and soaked thrice in TBST for 2 min. An anti-fluorescence quenching mounting medium was added dropwise, and the cover glass was mounted. The stained tissue sections were observed under a confocal microscope and analyzed.

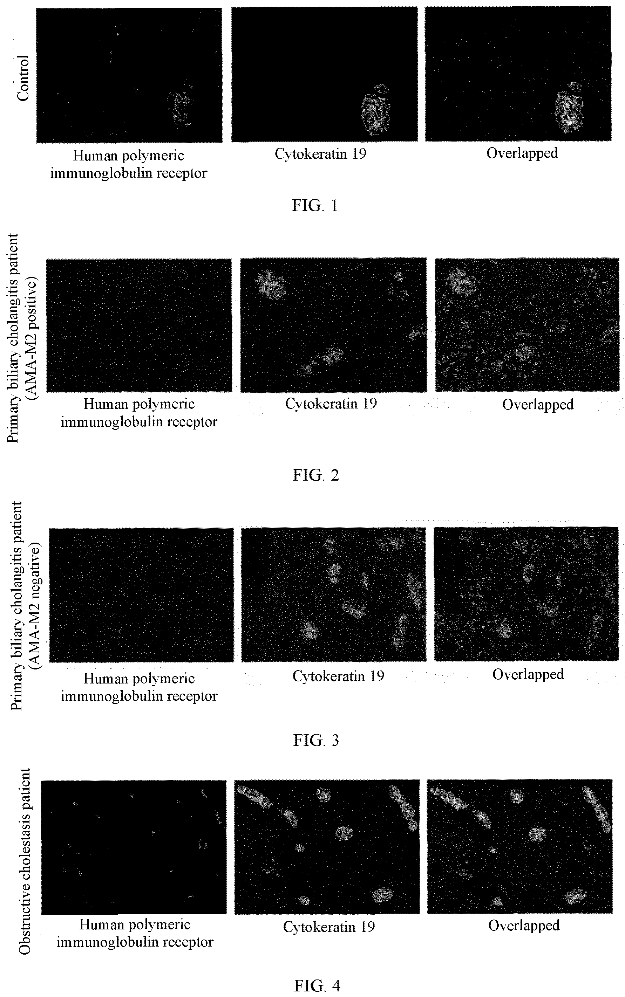

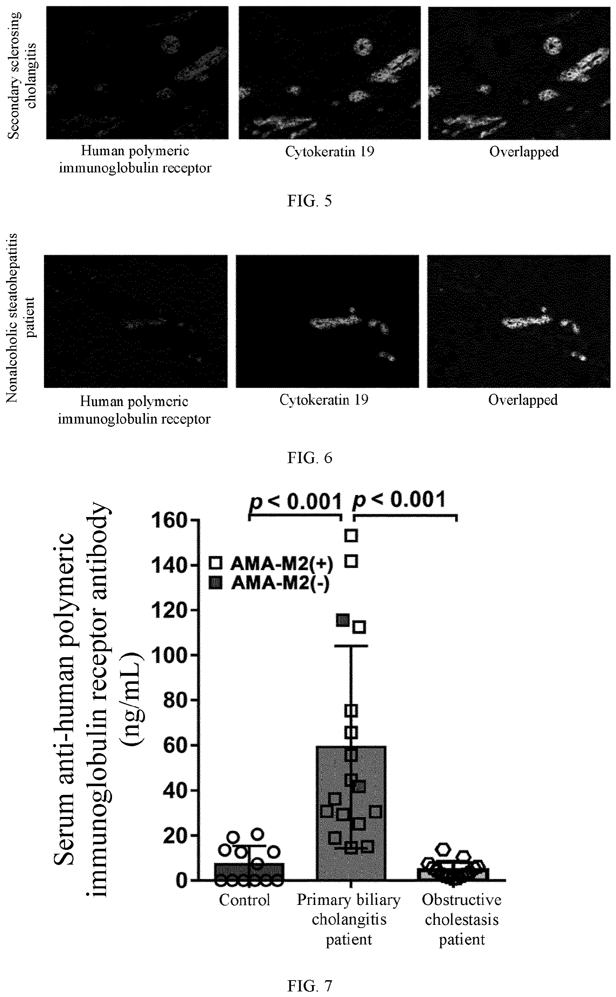

By the above procedures, the results demonstrate that the expression of human polymeric immunoglobulin receptor in small and medium-sized bile ducts in the liver of primary biliary cholangitis patients, both AMA-M2 positive (FIG. 2) or negative (FIG. 3), is all significantly reduced as compared with the control (FIG. 1), while in obstructive cholestasis patients (FIG. 4), secondary sclerosing cholangitis patients (FIG. 4), and nonalcoholic steatohepatitis patients (FIG. 6), human polymeric immunoglobulin receptor is expressed in biliary epithelial cells, which is similar to the control.

Example 2: Confirmation of expression of serum anti-human polymeric immunoglobulin receptor antibodies in CTR subjects and PBC and OC patients.

Peripheral blood samples were collected from 12 CTR subjects, 17 PBC patients, and 15 OC patients in the inpatient department of the Southwest Hospital. The expression of anti-human polymeric immunoglobulin receptor antibody in the serum of the subjects was detected by enzyme-linked immunosorbent assay (ELISA) using a human polymeric immunoglobulin receptor antibody ELISA kit (LunChangShuoBiotech, 100712). The specific procedures are as follows:

### (1) Serum collection:

A whole blood sample collected in a serum separation tube was let stand at room temperature for 2 h or at 4 °C overnight, and then centrifuged at 1000× g for 20 min to give a supernatant. The supernatant can be optionally preserved at -20 °C or -80 °C to avoid repeated freezing and thawing.

### (2) Preparation of reagents and instruments:

microplate, standard, sample diluent, detection antibody-HRP, 20× washing buffer, substrate A, substrate B, stop solution, sealing film, microplate reader (450 nm), high-precision pipette, and pipette tips: 0.5-10 µL, 2-20 µL, 20-200 µL, and 200-1000 µL, 37 °C thermostat, distilled water or deionized water.
Dilution of 20× washing buffer: The washing buffer was diluted with distilled water in a factor of 1:20, i.e., 1 part of the 20× washing buffer solution with 19 parts of distilled water added.

### (3) Procedures

The plates were equilibrated in an aluminum foil bag at room temperature for 20 min before use. The remaining plates were sealed in a re-sealable bag and preserved at 4 °C.

Standard wells and sample wells were set. 50 µL of standards of different concentrations were added into the standard wells, and 50 µL of the detection sample was added into the sample wells. Nothing was added into the blank wells.

100 µL of the detection antibody (the mouse anti-human polymeric immunoglobulin monoclonal antibody) labeled with horseradish peroxidase (HRP) was added into each of the standard wells and the sample wells. The reaction wells were sealed with the sealing film. The samples were incubated at 37 °C for 60 min in a water bath or thermostat.

The liquid was discarded. The plate was dried. Each well was filled with the washing solution (350 µL) and let stand for 1 min. The washing solution was removed and the plate was dried. The plate washing procedure was repeated 5 times (or the plate could be washed with a plate washer).

The substrates A and B, each 50 µL, were added into each well and incubated at 37 °C away from light for 15 min.

50 µL of the stop solution was added into each well, and the OD value of each well was measured at a wavelength of 450 nm within 15 min.

### (4) Calculation of results

A standard curve of the measured OD values of the standard vs the concentration of the standard was plotted, and a linear regression equation was obtained. The OD value of the sample was introduced into the equation to calculate the concentration of the sample.

By the above procedures, the results demonstrate that compared with the control subjects and the obstructive cholestasis patients, the anti-human polymeric immunoglobulin receptor antibody in the serum of the primary biliary cholangitis patients, both AMA-M2 positive and negative, is significantly increased (FIG. 7). This finding suggests that the anti-human polymeric immunoglobulin receptor antibody mediates the damage of cholangiocytes by targeting the human polymeric immunoglobulin receptor antigen on the small and medium-sized bile ducts in the liver, and serum anti-human polymeric immunoglobulin receptor antibodies can thus be used as a molecular marker for diagnosing primary biliary cholangitis.

The detailed description above illustrates preferred examples of the present invention. It should be appreciated that numerous modifications and changes can be made by those of ordinary skills in the art according to the concept of the present invention without creative efforts.

## Claims

1. Use of anti-human polymeric immunoglobulin receptor antibody in a sample obtained from a patient as a molecular marker for diagnosing primary biliary cholangitis (PBC).

2. The use according to claim 1, wherein the sample is anti-AMA-M2 antibody-positive or anti-AMA-M2 antibody-negative.

3. The use according to claim 1, wherein a reagent is used for detecting an expression level of the anti-human polymeric immunoglobulin receptor antibody.

4. The use according to claim 1, wherein the sample is a human serum sample.

5. The use according to claim 3, wherein the reagent is an enzyme-linked immunosorbent assay (ELISA) kit.

6. The use according to claim 5, wherein the ELISA kit comprises a plate, a standard, a sample diluent, a mouse anti-human polymeric immunoglobulin monoclonal antibody labeled with horseradish peroxidase (HRP), wherein the mouse anti-human polymeric immunoglobulin monoclonal antibody is a detection antibody, a 20× washing buffer, a substrate A, a substrate B, a stop solution, a sealing film, and a re-sealable bag.

7. The use according to claim 6, wherein using the ELISA kit comprises:
separating a serum from a whole blood sample as a detection sample;
setting standard wells and sample wells, adding 50 µL of standards of different concentrations into the standard wells, and adding 50 µL of the detection sample into the sample wells; adding nothing into blank wells;
adding 100 µL of the detection antibody labeled with HRP into each of the standard wells and the sample wells, sealing reaction wells with the sealing film, and incubating at 37 °C for 60 min;
washing the plate with a washing solution 5 times;
adding the substrates A and B, each 50 µL, into each well and incubating at 37 °C away from light for 15 min;
adding 50 µL of the stop solution into each well, and measuring optical density (OD) values of the wells at a wavelength of 450 nm within 15 min; and
plotting a standard curve of the measured OD values of the standard vs the concentration of the standard, obtaining a linear regression equation, and introducing the OD value of the sample into the linear regression equation to calculate the concentration of the sample.

## Patentansprüche

1. Verwendung eines Antikörpers gegen den humanen polymeren Immunoglobulinrezeptor in einer von einem Patienten gewonnenen Probe als molekularem Marker zum Diagnostizieren von primär biliärer Cholangitis (PBC).

2. Verwendung nach Anspruch 1, wobei die Probe Anti-AMA-M2-Antikörper-positiv oder Anti-AMA-M2-Antikörper-negativ ist.

3. Verwendung nach Anspruch 1, wobei ein Reagenz zum Nachweis eines Expressionsniveaus des Antikörpers gegen den humanen polymeren Immunoglobulinrezeptor verwendet wird.

4. Verwendung nach Anspruch 1, wobei die Probe eine menschliche Serumprobe ist.

5. Verwendung nach Anspruch 3, wobei das Reagenz ein enzymgekoppelter Immunadsorptionstest(ELISA-)Kit ist.

6. Verwendung nach Anspruch 5, wobei das ELISA-Kit eine Platte, eine Standardlösung, ein Probenverdünnungsmittel, einen monoklonalen Maus-Antikörper gegen humanes polymeres Immunoglobulin, markiert mit Meerrettichperoxidase (HRP), wobei der monoklonale Maus-Antikörper gegen humanes polymeres Immunoglobulin ein Nachweisantikörper ist, einen 20-fachen Waschpuffer, ein Substrat A, ein Substrat B, eine Stopplösung, eine Versiegelungsfolie und eine wiederverschließbare Tasche umfasst.

7. Verwendung nach Anspruch 6, wobei das Verwenden des ELISA-Kits Folgendes umfasst:
Trennen eines Serums aus einer Vollblutprobe als Nachweisprobe;
Festlegen von Referenzvertiefungen und Probenvertiefungen, Hinzufügen von 50 µl Standardlösungen unterschiedlicher Konzentrationen in die Standardvertiefungen und Hinzufügen von 50 µl der Nachweisprobe in die Probenvertiefungen; Hinzufügen von nichts in die Blindvertiefungen;
Hinzufügen von 100 µl des mit HRP markierten Nachweisantikörpers in jede der Referenzvertiefungen und der Probenvertiefungen, Versiegeln der Reaktionsvertiefungen mit der Versiegelungsfolie und 60-minütiges Inkubieren bei 37 °C;
5-maliges Waschen der Platte mit einer Waschlösung;
Hinzufügen der Substrate A und B, jeweils 50 µl, in jede Vertiefung und 15-minütiges Inkubieren bei 37 °C vor Licht geschützt;
Hinzufügen von 50 µl der Stopplösung in jede Vertiefung und Messen der Werte der optischen Dichte (OD) der Vertiefungen bei einer Wellenlänge von 450 nm innerhalb von 15 min. und
Aufzeichnen einer Standardkurve der gemessenen OD-Werte der Standardlösung gegenüber der Konzentration der Standardlösung, Erlangen einer linearen Regressionsgleichung und Einführen des OD-Werts der Probe in die lineare Regressionsgleichung, um die Konzentration der Probe zu berechnen.

## Revendications

1. Utilisation d'un anticorps anti-récepteur d'immunoglobuline multimérique humaine dans un échantillon obtenu à partir d'un patient en tant que marqueur moléculaire pour le diagnostic de la cholangite biliaire primitive (PBC).

2. Utilisation selon la revendication 1, dans laquelle l'échantillon est positif à un anticorps anti-AMA-M2 ou négatif à un anticorps anti-AMA-M2.

3. Utilisation selon la revendication 1, dans laquelle un réactif est utilisé pour détecter un niveau d'expression de l'anticorps anti-récepteur d'immunoglobuline multimérique humaine.

4. Utilisation selon la revendication 1, dans laquelle l'échantillon est un échantillon de sérum humain.

5. Utilisation selon la revendication 3, dans laquelle le réactif est un kit d'immunoadsorption par enzyme liée (ELISA).

6. Utilisation selon la revendication 5, dans laquelle le kit ELISA comprend une plaque, un standard, un diluant d'échantillon, un anticorps monoclonal de souris anti-immunoglobuline multimérique humaine marqué avec de la peroxydase de raifort (HRP), dans laquelle l'anticorps monoclonal de souris anti-immunoglobuline multimérique humaine est un anticorps de détection, un tampon de lavage 20x, un substrat A, un substrat B, une solution d'arrêt, un film d'étanchéité et un sac refermable.

7. Utilisation selon la revendication 6, dans laquelle l'utilisation du kit ELISA comprend :
la séparation d'un sérum d'un échantillon de sang total en tant qu'échantillon de détection ;
le réglage de puits standard et de puits d'échantillon, l'ajout de 50 µL de standards de différentes concentrations dans les puits standard, et l'ajout de 50 µL de l'échantillon de détection dans les puits d'échantillon ; ne rien ajouter dans les puits vierges ;
l'ajout de 100 µL de l'anticorps de détection marqué avec de la HRP dans chacun des puits standard et des puits d'échantillon, le scellement des puits de réaction avec le film d'étanchéité, et l'incubation à 37°C pendant 60 min ;
le lavage de la plaque avec une solution de lavage 5 fois ;
l'ajout des substrats A et B, chacun de 50 µL, dans chaque puits et l'incubation à 37°C à l'abri de la lumière pendant 15 min ;
l'ajout de 50 µL de la solution d'arrêt dans chaque puits, et la mesure des valeurs de la densité optique (OD) des puits à une longueur d'onde de 450 nm dans les 15 min ; et
le tracé d'une courbe standard des valeurs OD mesurées du standard par rapport à la concentration du standard, l'obtention d'une équation de régression linéaire, et l'introduction de la valeur OD de l'échantillon dans l'équation de régression linéaire pour calculer la concentration de l'échantillon.
